# EUROPEAN PATENT APPLICATION

(11) **EP 3 599 297 A1**
(43) Date of publication of application: **29.01.2020**
(21) Application number: 18185628.7
(22) Date of filing: 25.07.2018
(51) Int. Cl.: D01F 6/62, D01F 8/14, D01D 5/00, D01D 5/18

(54) **COMPOSITE SYSTEM BASED ON FUNCTIONALIZED NANOFIBERS FOR PROTECTION OF ACTIVE SUBSTANCES IN LACQUERS**

(71) Applicant: Nanuntio s.r.o., 16000 Prague 6 (CZ)
(72) Inventor: Amler, Evzen, 17000 Prague 7 (CZ); Zavodska, Jana, 37701 Jindrichuv Hradec (CZ); Zavodska, Jana, 37701 Jindrichuv Hradec (CZ); Pokorny, Ivan, 25001 Brandys nad Labem (CZ)
(74) Representative: Novotny, Jaroslav

(57) **Abstract**

Composite system for temporary protection of the active substance against solvent in nail vanishes and lacquers during drying process characterized by encapsulation of an active substance into nanofibers prepared from PCL or a polymers with a shorter degradation halftime than is halftime of lacquer drying followed by fractionalization or micro fractionalization.

## Description

### Technical Fields

Invention deals with a way of protection of active substances at lacquers, namely at nail vanishes, their subsequent release and due to their presence an improvement of mechanical parameters of lacquers and nail vanishes. Active substance protection is achieved by their encapsulation into core of microsystems produced either microfractionalization from nanofibers or by electrospraying.

### Background Arts

There are several reports and patents for esthetic, functional and mechanical improving of lacquers. Closest situation is described, for example, at EP 2012-808365 on application of additives improving engine lacquers, at EP 2010-709427 on application of copolymers as additives to lacquers, as well as at PV 1993-1753 on polyurethane resin dispersion in aqueous solutions for coating, or at PV 2015-646 lacquer with antimicrobial culture.

However, all these improvements and solutions deal with additives and active substance which are slightly influenced, if at all, when mixed with solvent. There is, however, a huge demand on distribution of intact active substances and additives also in the case of their possible direct interaction with solvents. In addition, even an aggregation and not necessarily any chemical reaction or interaction, could significantly negatively influenced the potential application, namely from the esthetic point of view. These esthetic and functional shortcomings limit application of additives into lacquers, namely nail vanishes.

### Disclosure of Invention

Our composite system of functionalized nanofibers protects active substances in lacquers and minimizes or eliminates the above mentioned shortcomings. The principle of invention is, first, at encapsulation of active substances soluble or interacting with solvent into nanofibers with a lower solubility in solvent, typically polycaprolactone (PCL), by electrospinning process or by forcespinning. This process can be followed by microfractionalization to achieve a fraction of desired size and to control better the releasing process. Nanofibers could also be with an advantage prepared from suspension followed by fractionalization or microfractionalization. Active substance to a nail vanish could be encapsulated into nanofibers by electrospraying or electrospinning followed by microfractionalization. Active substance to lacquers is encapsulated by any of the above mention process into nanofibers prepared from polymers with a degradation half-time slower than a half-time of lacquer drying. The process of encapsulation is followed by microfractionalization, if needed, typically in liquid nitrogen, to obtain a powder-like substance as an additive with internal nano- and micro-structure.

Application of this invention is characterized with distribution of additives with:
a. Minimal degradation or other damage of active substances
b. Prolongation of the additive active effect
c. Minimal aggregation process which namely at nail vanishes contributes to a higher esthetic level
d. Improved mechanical parameters of lacquers and vanishes.

Protection of active additives against degradation processes in solvents and improvement mechanical properties of lacquers and nail vanishes is achieved according to this invention by:
a. Encapsulation spherical nanosystems by electrospraying
b. Encapsulation of active additives into nanofibers followed by microfractionalization
c. Emulsification of active additives on the interface of two unmixable systems

### Made for Carrying out the Invention

### Example 1

Additive to a nail vanish is an active substance with a low solubility in PCL enncapsulated into nanofiber core by the method of coaxial spinning followed by nanofiber fractionalization or microfractionalization.

### Example 2

Additive to a nail vanish is an active substance with a low solubility in PCL enncapsulated into nanofiber core by the method of spinning frpm suspension followed by nanofiber fractionalization or microfractionalization.

### Example 3

Additive to a nail vanish is an active substance with a low solubility in PCL enncapsulated into nanofiber core by the method of cetrifugal spinning followed by nanofiber fractionalization or microfractionalization.

### Example 4

Additive to a nail vanish is an active substance with a low solubility in PCL enncapsulated into nanofiber core by the method of electrospraying.

### Example 5

Additive to lacquers for improvement their mechanical and functional parameters incorporated into nanofibers by any of above mentioned Examples. Nanofibers are fabricated from polymer or polymers with halftime of solubility in lacquer solvent shorter than time of drying

### Industrial Application of the Invention

The invention is applicable for protection of active substances used as additives to lacquers, namely to nail vanish, and their gradual release with a simultaneous improvement of lacquer mechanical parameters. Protection and functional improvement is achieved by active substance encapsulation into microsystems prepared from nanofibers or as a consequence of electrospraying.

## Claims

1. Composite system on a nanofiber basis for protection of active substances in nail vanish **characterized by** encapsulation of an active substance changing its characteristics when in contact with solvents, into PCL nanofibers by coaxial spinning followed by fractionalization or microfractionalization.

2. Composite system on a nanofiber basis for protection of active substances in nail vanish **characterized by** encapsulation of an active substance changing its characteristics when in contact with solvents, into PCL nanofibers by spinning from suspension followed by fractionalization or microfractionalization.

3. Composite system on a nanofiber basis for protection of active substances in nail vanish **characterized by** encapsulation of an active substance changing its characteristics when in contact with solvents, into PCL nanofibers by force spinning followed by fractionalization or microfractionalization.

4. Composite system on a nanofiber basis for protection of active substances in nail vanish **characterized by** encapsulation of an active substance changing its characteristics when in contact with solvents, into PCL nanofibers by electrospraying.

5. Composite system on a nanofiber basis for protection of active substances in lacquers **characterized by** encapsulation of an active substance changing its characteristics when in contact with solvents, into nanofibers prepared by coaxial spinning, suspension spinning, force spinning or centrifugal spinning from a polymers with a shorter degradation halftime than is halftime of lacquer drying followed by fractionalization or microfractionalization.
